# EUROPEAN PATENT SPECIFICATION

(11) **EP 4 119 536 B1**
(45) Date of publication and mention of the grant of the patent: **14.08.2024**
(21) Application number: 22185148.8
(22) Date of filing: 15.07.2022
(51) Int. Cl.: C07C 45/46, C07C 49/675, C07C 209/68, C07C 209/62, C07C 213/00, C07C 211/32, C07C 215/42, C07C 249/08, C07C 251/58

(54) **PREPARATION OF A PHARMACEUTICAL INTERMEDIATE**
HERSTELLUNG EINES PHARMAZEUTISCHEN ZWISCHENPRODUKTS
PRÉPARATION D'UN PRODUIT PHARMACEUTIQUE INTERMÉDIAIRE

(30) Priority: 15.07.2021 IT 202100018758
(43) Date of publication of application: 18.01.2023
(73) Proprietor: Dipharma Francis S.r.l., 20021 Baranzate (MI) (IT)
(72) Inventor: DELLA PENNA, Francesca, 67100 L'AQUILA (AQ) (IT); NORI, Valeria, 67100 L'AQUILA (AQ) (IT); PESCIAIOLI, Fabio, 67100 L'AQUILA (AQ) (IT); CARLONE, Armando, 67100 L'AQUILA (AQ) (IT)
(74) Representative: Bianchetti & Minoja with Trevisan & Cuonzo IPS SRL

(56) References cited:
- WO-A1-2013/168008
- GB-A- 1 045 911
- TAKEHIKO YAMATO ET AL: "Organic reactions catalyzed by solid superacids. 5. Perfluorinated sulfonic acid resin (Nafion-H) catalyzed intramolecular Friedel-Crafts acylation", THE JOURNAL OF ORGANIC CHEMISTRY, vol. 56, no. 12, 1 June 1991 (1991-06-01), pages 3955 - 3957, XP055048076, ISSN: 0022-3263, DOI: 10.1021/jo00012a033
- HUDGENS D P ET AL: "Discovery of diphenyl amine based sodium channel blockers, effective against hNa"v1.2", BIOORGANIC, ELSEVIER, AMSTERDAM, NL, vol. 14, no. 24, 15 December 2006 (2006-12-15), pages 8366 - 8378, XP027993041, ISSN: 0968-0896, [retrieved on 20061215]
- OLAH GEORGE A ET AL: "Nafion-H Catalysed Intramolecular Friedel-Crafts Acylation: Formation of Cyclic Ketones and Related Heterocycles 1", 1 July 1999 (1999-07-01), pages 1067 - 1068, XP055908630, Retrieved from the Internet <URL:https://www.thieme-connect.com/products/ejournals/pdf/10.1055/s-1999-2770.pdf>
- BLAESS MARKUS ET AL: "NB 06: From a simple lysosomotropic aSMase inhibitor to tools for elucidating the role of lysosomes in signaling apoptosis and LPS-induced inflammation", EUROPEAN JOURNAL OF MEDICINAL CHEMISTRY, vol. 153, 1 June 2018 (2018-06-01), AMSTERDAM, NL, pages 73 - 104, XP093000967, ISSN: 0223-5234, DOI: 10.1016/j.ejmech.2017.09.021
- BLAESS MARKUS ET AL: "NB 06: From a simple lysosomotropic aSMase inhibitor to tools for elucidating the role of lysosomes in signaling apoptosis and LPS-induced inflammation", EUROPEAN JOURNAL OF MEDICINAL CHEMISTRY, vol. 153, 1 June 2018 (2018-06-01), AMSTERDAM, NL, pages 73 - 104, XP093000967, ISSN: 0223-5234, DOI: 10.1016/j.ejmech.2017.09.021

## Description

### FIELD

The present invention concerns a new process for preparing dibenzosuberone, a key intermediate in the synthesis of active pharmaceutical ingredients useful in the treatment of diseases related to the central nervous system.

### STATE OF THE ART

Dibenzosuberone of formula **(I)** 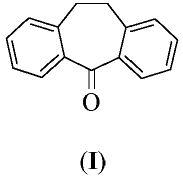 is a key intermediate in the synthesis of several tricyclic antidepressant drugs, commonly known as tricyclic, or abbreviated TCA, such as amitriptyline, nortriptyline or noxiptiline.

The compound of formula **(I)** is generally prepared by intramolecular Friedel-Crafts acylation reaction of 2-(phenethyl)benzoic acid of formula **(II)**

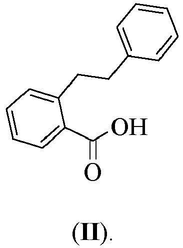

Due to the low reactivity of carboxylic acids, they often have to be activated by converting them into the corresponding acyl chloride. Nevertheless, at least stoichiometric quantities of a Lewis acid, for example AlCl₃, are furthermore required to obtain dibenzosuberone of formula **(I)**. Consequently, it would be desirable to develop an efficient, economical and sustainable process, which just makes use of activating systems in catalytic quantities.

Despite the considerable efforts made so far to render the Friedel-Crafts reaction more efficient and environmentally friendly, even current industrial processes not only use much more than stoichiometric quantities of Lewis acids as activating agents, but also make use of considerable quantities of chlorinated solvents, in particular dichloromethane, dichloroethane or chlorobenzenes.

Alternatively, dibenzosuberone of formula **(I)** can be obtained by cyclization of 2-(phenethyl)benzoic acid of formula **(II)** with polyphosphoric acid (PPA), which again is used in more than stoichiometric quantities as an activating agent.

PPA is a good solvent for organic compounds, it can be used at high temperatures and it is not excessively acidic. However, it is a highly viscous and hygroscopic liquid.

In addition, at the end of the process, it is necessary to dilute the PPA as well as the Lewis acids with large quantities of water to recover the desired product and, before eliminating the waste water, it is necessary to neutralize the acidity by adding an appropriate base. This neutralization results in the formation of high quantities of salts, which must be then disposed of.

Yamato, T. et al. reports in J. Org. Chem. 1991, 56, n. 12, 3955-3957 in Table 1 that the compound of formula **(II)** catalyzed by Nafion-H in para-xylene fully converts into dibenzosuberone of formula **(I)** after 12 hours. However, the authors reported that unexpectedly no product was detected in benzene: even after 36 hours of reaction only the starting material was identified.

For these reasons, there is still a need to have an industrial process for preparing dibenzosuberone of formula **(I)**, which makes use of solvents, which can be easily removed from the reaction mixture and which are non-toxic, and which requires catalytic quantities of activating agents. Said desired process has to be economical, both in respect to the cost of the reagents and in respect to the treatment of waste water, it has to be eco-compatible and should allow an easy recycling of the solvents, catalysts and/or activating agents used for the cyclisation. Such method should also encompass mild reaction conditions and at the same time provide the desired compound in high yields and at high purity.

### SUMMARY

An object of the present invention is directed to a process for preparing a compound of formula **(I)** 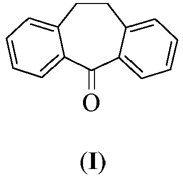 comprising cyclizing the compound of formula **(II)** 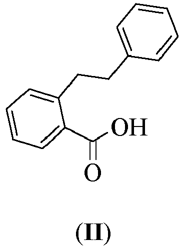 in presence of a sulfonated styrene-divinylbenzene copolymer ion exchange resin.

### DETAILED DESCRIPTION

An object of the present invention is directed to a process for preparing a compound of formula **(I)** 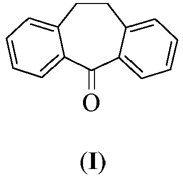 comprising cyclizing the compound of formula **(II)** 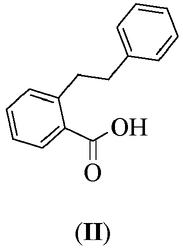 in presence of a sulfonated styrene-divinylbenzene copolymer ion exchange resin.

According to the present invention, the term "comprising" means that additional reaction steps may be present, but which do not substantially modify the product obtained from the process. The term "comprising" also includes the terms "consisting" and "essentially consisting of".

The compound of formula **(II)** is a known compound and is commercially available. For example, the compound of formula **(II)** is sold by ABCR (https://abcr.com, catalogue number: AB149010).

Alternatively, the compound of formula **(II)** can be prepared according to known methods, for example by treating benzphthalide with dipentene in the presence of palladium on carbon (Ramesha AR. et al. in Synthetic Communications 2001, 31, 2419-2422).

The sulfonated styrene-divinylbenzene copolymer ion exchange resin is a polymeric ion exchange resin with acidic characteristics and that can be used as a heterogeneous acid catalyst. The ratio between the two components of the copolymer provides different surface and porosity characteristics.

The copolymer can be a gel or a macroreticular (macroporous) composition with spherical particles having generally a diameter between about 0.3 and about 1.2 millimeters, but it can be also larger or smaller.

According to the present invention, the term "ionic" includes cationic or anionic.

In a preferred aspect of the invention, the ion exchange resin is a cation exchange resin.

In a preferred aspect of the invention, the copolymer is in macroreticular form with spherical particles with a diameter of about 0.3 to about 1.2 millimeters, or smaller.

Examples of commercially available copolymers are Amberlyst, in particular Amberlyst^{®} 15 (CAS code 39389-20-3, catalogue code 89079, Alfa Aesar), Amberlyst^{®} XN-1010 (catalogue code 216402, Sigma Aldrich), Amberlyst^{®} 35 (catalogue code 45-D00929, Dupont), Amberlyst^{®} 36 (catalogue code 45-D00930, Dupont), Amberlyst^{®} 46 (Lenntech, a Rohm and Haas company), Amberlyst^{®} 70 (Lenntech, a Rohm and Haas company), Amberlite^{®} IRC120 H (catalogue code 06428, Sigma Aldrich) or Purolite CTE275^{®} (Purolite International Limited, Rhondda Cynon Taff, Wales, UK).

In a more preferred aspect of the invention, the sulfonated styrene-divinylbenzene copolymer ion exchange resin is chosen from Amberlyst^{®} 15, Amberlyst^{®} XN-1010, Amberlyst^{®} 35, Amberlyst^{®} 36, Amberlyst^{®} 46, Amberlyst^{®} 70 or Purolite CTE275^{®}., more preferably from Amberlyst^{®} 15, Amberlyst^{®} 36, or Purolite CTE275^{®}.

The sulfonic acid/styrene-divinylbenzene ratio can be equimolar, as for example for Amberlyst resin^{®} 15, higher than one, as in Amberlyst resins^{®} 35 or Amberlyst^{®} 36, or less than one, as for Amberlyst resin^{®} 70.

The procedure for obtaining the compound of formula **(I)** using the sulfonated styrene-divinylbenzene copolymer ion exchange resin can be carried out, for example, by a continuous flow process, for instance in a packed bed reactor, or by a batch process.

According to one aspect of the invention, the process may be carried out by a continuous flow process, for instance in a packed bed reactor.

According to one aspect of the invention, the process may be carried out continuously by passing a suspension of the ion exchange resin in the reactants through a reaction zone, where it is heated at the reaction temperature.

According to one aspect of the invention, the process may be carried out continuously by passing the reactants in a suspension of the ion exchange resin, where it is heated at the reaction temperature.

The herein disclosed ion exchange resin does not only allow to carry out the cyclization reaction of 2-(phenethyl)benzoic acid of formula **(II)** without the need of preparing the corresponding activated acyl chloride, but also does not require a Lewis acid, such as AlCl₃, or PPA. The resins can be also easily filtered off from the reaction mixture and they can be regenerated after appropriate washing and reused for multiple reaction cycles. By this way, it is possible to reduce the amount of the produced waste and at the same time to recover the costs by reusing the catalyst.

A first use of a cation exchange resin for preparing dibenzosuberone of formula **(I)** was reported by Olah, G.A. et al. in Synlett, 1999, 7, 1067-1068, "Nafion-N Catalysed Intramolecular Friedel-Crafts Acylation: Formation of Cyclic Ketones and Related Heterocycles 1". In particular, Olah *et al.* have studied Nafion-H^{®} resins, which are considered super-acidic resins having an acidity higher than 100% pure sulfuric acid, and which are obtained by copolymerization of tetrafluoroethylene (TFE, C₂F₄) (Teflon) with a perfluorinated sulfonic acid derivative, in specific perfluoro-3,6-dioxa-4-methyl-7-octenesulfonic acid fluoride. However, Olah *et al.* observed that the reaction with Nafion-H^{®} resins as catalysts does not lead to the desired product in low boiling solvents, such as dichloromethane or CCl₄. On the other hand, using high-boiling solvents such as 1,2-dichlorobenzene (boiling point of 180°C) and 1,3-dichlorobenzene (boiling point of 173°C) and maintaining the reaction mixture at reflux for 3 hours allowed to obtain dibenzosuberone of formula **(I)** with yields of 95%. However, such solvents are difficult to be removed from the reaction mixture.

Olah *et al.* furthermore stated that several other side products are formed in toluene or xylene, for example due to intermolecular reactions. These results are in contrast to a precedent publication by the same Olah group (Yamato, T. et al. in J. Org. Chem. 1991, 56, n. 12, 3955-3957, "Organic reactions catalyzed by solid superacids. 5. Perfluorinated sulfonic acid resin (Nafion-H) catalysed intramolecular Friedel-Crafts acylation"): this *J*. *Org. Chem.* paper reports in Table 1 that the compound of formula **(II)** catalyzed by Nafion-H in para-xylene fully converts into dibenzosuberone of formula **(I)** after 12 hours. However, the authors reported that unexpectedly no product was detected in benzene: even after 36 hours of reaction only the starting material was identified.

The Nafion-H resins used in the experiments described in both publications were prepared in house in the Olah laboratory, rendering impracticable a precise reproduction of the disclosed experiments.

As an additional note, commercially available Nafion-H^{®} resin is supplied in the form of sheets that must be cut before being added to the reaction mixture. Furthermore, its recovery after completion of the reaction is not straightforward, which renders the use of the resin problematic for an industrial application.

For this reason, and especially due to the difficult removal of 1,2-dichlorobenzene and 1,3-dichlorobenzene from the reaction mixture, the procedures described by Olah et al. in Synlett 1999, 7, 1067-1068 are hardly applicable on an industrial scale.

The inventors of the present application have surprisingly found an industrial process for preparing dibenzosuberone of formula **(I)** that overcomes the difficulties encountered with Nafion-H^{®} resins by using ion exchange resins based on a sulfonated styrene-divinylbenzene copolymer, even though they exhibit a substantially lower acidity. These findings are particularly surprising, since Olah G. A. and Prakash G. K. S. et al. reported in Arkivoc, 2004, (viii), 103-111, "Aroylation of aromatics with arylcarboxylic acids over Nafion-H (polymeric perfluoroalkanesulfonic acid), an environmentally friendly solid acid catalyst, a later publication than the above-mentioned two publications in *Synlett* and *J*. *Org. Chem.*, that "*only extremely reactive aromatics such as thiophenes and substituted thiophenes have been successfully acylated using solid catalysts such as sulfonated polystyrene resins*"*.* In fact, US 2,711,414, to which the article refers, discloses that sulphonated copolymers of monovinyl- and polyvinyl-aromatic hydrocarbons have been found to be able to catalyze acylation reactions on thiophene derivatives solely at very specific reaction conditions.

If appropriate, the reaction can be carried out in a solvent. This solvent can then be recycled by distillation at the end of the reaction.

In one aspect of the invention, the solvent is an organic solvent having a boiling temperature between about 0°C and about 160°C. This solvent allows to obtain dibenzosuberone of formula **(I)** at high yields even at temperatures lower than those employed by Olah *et al..*

In one aspect of the invention, the organic solvent is typically an aprotic apolar organic solvent having a boiling temperature between about 0°C and about 160°C, and is typically a C₄-C₁₂ alkane, an aromatic hydrocarbon, a chlorinated solvent, an ethereal solvent, a dipolar aprotic solvent; or a mixture of two or more, for example two or three, of the solvents mentioned above.

According to the present invention, a C₄-C₁₂ alkane refers to a linear, branched or cyclic hydrocarbon chain, consisting only of carbon and hydrogen atoms and having four to twelve carbon atoms. Examples of a C₄-C₁₂ alkane are butane, pentane, n-pentane, hexane, n-hexane, heptane, n-heptane or cyclohexane.

According to the present invention, aromatic hydrocarbons refer to hydrocarbons derived from benzene. Examples of an aromatic hydrocarbon are toluene, ortho-xylene, meta-xylene, or para-xylene.

According to the present invention, a chlorinated solvent is for example dichloromethane (CH₂Cl₂), chloroform, 1,1-dichloroethane or 1,2-dichloroethane.

According to the present invention, an ethereal solvent is typically tetrahydrofuran (THF), methyl *tert*-butyl ether (MTBE), methyl-THF or dioxane.

According to the present invention, a dipolar aprotic solvent is typically dimethylformamide, dimethylacetamide, acetonitrile, 1-methyl-2-pyrrolidone (or *N-*methyl-2-pyrrolidone or NMP) or dimethyl sulfoxide (DMSO).

In a preferred aspect of the invention, the organic solvent is chosen from hexane, heptane, cyclohexane, toluene, ortho-xylene, meta-xylene, para-xylene or a mixture of two or more, for example two or three, of the above-mentioned solvents. Therefore, if desired, the use of chlorinated solvents, such as chlorobenzenes, can be avoided.

According to a further aspect of the present invention, the cyclization reaction can be carried out without the use of any solvent, for example by melting the compound of formula **(II)**, which has a melting point around 130°C at ambient pressure, and by adding the ion exchange resin.

The cyclization reaction of the compound of formula **(II)** can be carried out at a temperature between about 0°C and the reflux temperature of the reaction mixture, for example at a temperature between 0°C and 160°C. Typically, the reaction can be carried out at about 150°C, at about 140°C, at about 130°C, at about 120°C, at about 110°C, at about 100°C, at about 90°C, at about 80°C, at about 70°C, at about 60°C, at about 50°C or at about 40°C.

In a preferred aspect of the invention, the cyclization reaction of the compound of formula **(II)** can be carried out at a temperature between 0°C and 150°C. More preferably, the reaction can be carried out at about 150°C, at about 140°C, at about 130°C, at about 120°C, at about 110°C, at about 100°C, at about 90°C, at about 80°C, at about 70°C, or at about 60°C.

The cyclization reaction of the compound of formula **(II)** can be carried out at ambient pressure or under pressure, i.e. in a closed vessel. Typically, the reaction can be carried out at about ambient pressure, at about 2 bar, at about 3 bar, at about 4 bar, at about 5 bar, at about 7.5 bar, at about 10 bar, at about 20 bar, at about 30 bar, at about 40 bar, or at about 50 bar.

The cyclization reaction of the compound of formula **(II)** can be advantageously carried out in 1 hour or more, for example in 6 hours, 12 hours, 24 hours, 36 hours, 48 hours, 72 hours, 96 hours, 144 hours, 240 hours or in 480 hours.

The reaction can be advantageously performed by employing about 100.0 to about 0.001 moles of sulphonic acid groups of the sulfonated styrene-divinylbenzene copolymer per mole of compound of formula **(II)**, e.g. 10 moles, 5 moles, or 1 mole per mole of compound of formula **(II)**.

According to one aspect of the invention, the ion exchange resin can be used in smaller quantities, for example at 0.5 moles, 0.35 moles, 0.2 moles, 0.1 moles, 0.05 moles, or 0.01 moles of sulphonic acid groups per mole of compound of formula **(II)**.

According to a preferred aspect of the invention, the ion exchange resin can be used at 0.50 moles, 0.45 moles, 0.40 moles, 0.35 moles, 0.30 moles, 0.25 moles, 0.20 moles, 0.15 moles 0.10 moles, 0.05 moles, or 0.01 moles of sulphonic acid groups per mole of compound of formula **(II)**.

According to one aspect of the invention, the reactants and the ion exchange resin may be admixed with one another in any desired order.

In a further aspect of the invention, the compound of formula **(II)** can be activated to form a compound of formula **(III)** 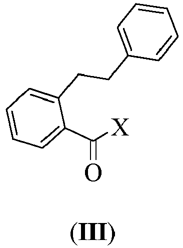 wherein **X** is a halogen or a O-(C=O)-**R** group,
and wherein **R** is chosen from C₁-C₆ alkyl, C₃-C₈ cycloalkyl, C₁-C₆ alkoxy or -O-(C=O)-imidazolyl,
and then treating the compound of formula **(III)** with the sulfonated styrene-divinylbenzene copolymer ion exchange resin.

According to the present invention, the halogen is chosen from fluorine, chlorine, bromine or iodine.

According to the present invention, the term "C₁-C₆ alkyl" refers to a linear or branched hydrocarbon chain, consisting only of carbon and hydrogen atoms and having one to six carbon atoms.

According to a preferred aspect of the present invention, the C₁-C₆ alkyl group is a linear or branched C₁-C₄ alkyl group. Examples are methyl, ethyl, n-propyl, *iso*-propyl, n-butyl, *sec*-butyl or *tert*-butyl.

According to the present invention, the term "C₃-C₈ cycloalkyl" refers to a cyclic hydrocarbon chain, consisting only of carbon and hydrogen atoms and having three to eight carbon atoms. Examples are cyclopropyl, cyclobutyl or cyclohexyl.

According to the present invention, the term "C₁-C₆ alkoxy" refers to a -O-C₁-C₆ alkyl group, wherein the C₁-C₆ alkyl group is as defined above.

In a preferred aspect of the invention, **X** is chlorine.

The inventors of the present invention have surprisingly found that the activation of the compound of formula **(II)** to the compound of formula **(III)** accelerates significantly the cyclization reaction of the compound of formula **(II)** to the compound of formula **(I)**.

The 2-(phenethyl)benzoic acid of formula **(II)** can be activated with an activating agent, e.g. with thionyl chloride (SOCl₂), thionyl bromide, oxalyl chloride, oxalyl bromide, phosphorus trichloride, phosphorus tribromide, phosphorus pentachloride, phosphorus pentabromide, a C₂-C₁₂ anhydride, such as acetic anhydride, or a reagent activated as di-*tert*-butyl dicarbonate or carbonyldiimidazole (CDI), optionally in presence of a solvent, wherein the solvent and the reaction conditions are as defined above.

The process can be advantageously carried out by employing about 10.0 to about 0.1 moles of the activating agent per mole of the compound of formula **(II)**, e.g. 9 moles, 8 moles, 7 moles, 6 moles, 5 moles, 4.5 moles, 4 moles, 3.5 moles, 3.3 moles, 3 moles, 2.7 moles, 2.5 moles, 2.2 moles, 2 moles, 1.5 moles, 1 moles, 0.7 moles, 0.7 moles or 0.3 moles of the activating agent per mole of the compound of formula **(II)**.

In a preferred aspect of the invention, the process can be advantageously carried out by employing about 1.1 to about 5.0 moles, more preferably from about 4.5 to about 1.5 moles, for example 2.0 moles, 2.5 moles, 2.7 moles, 3.0 moles, 3.3 moles, 3.5 moles, 3.7 moles or 4.0 moles of the activating agent per mole of compound of formula **(II)**.

According to one aspect of the invention, the compound of formula **(III)** can be obtained, for example, by treating 2-(phenethyl)benzoic acid of formula **(II)** with SOCl₂, optionally in the presence of a solvent, wherein the solvent is as defined above.

In a preferred aspect of the invention, the activation of the compound of formula **(II)** to the compound of formula **(III)** and subsequent conversion to the compound of formula **(I)** can be carried out at a temperature between 0°C and 160°C. More preferably, the process can be carried out at about 150°C, at about 140°C, at about 130°C, at about 120°C, at about 110°C, at about 100°C, at about 90°C, at about 80°C, at about 70°C, or at about 60°C.

The cyclization process of the compound of formula **(II)** can be advantageously carried out from about 10 minutes to 96 hours, for example in about 1 hour, 2 hours, 3 hours, 4 hours, 5 hours, 6 hours, 12 hours, 24 hours, 36 hours 48 hours or in about 72 hours.

In a preferred aspect of the invention, the reaction mixture comprising the compound of formula **(III)** can be used without further treatment, by adding the ion exchange resin, as defined above, in the same reactor, i.e. by carrying out a "one-pot reaction", in order to obtain a compound of formula **(I)** in two consecutive synthetic steps without intermediate isolation.

In a further preferred aspect of the invention, the activation of the compound of formula **(II)** to obtain a compound of formula **(III)** and its cyclization to the compound of formula **(I)** can be carried out substantially simultaneously, by treating a compound of formula **(II)** with the activating agent and the ion exchange resin, optionally in the presence of a solvent at the reaction conditions as defined above.

According to the present invention, the term substantially simultaneously means immediately subsequent or simultaneous activation and cyclization process.

At the end of the cyclization reaction, the workup of the reaction mixture can be performed according to methods well known to the skilled person in the art. The resin can be easily filtered off from the reaction mixture, regenerated by appropriate washing and reused for multiple reaction cycles. This reduces the amounts of waste produced and has the advantage of amortizing costs by reusing the catalyst.

The so obtained crude reaction product of formula **(I)** can be further purified by known methods.

For example, the reaction mixture can be concentrated, optionally at reduced pressure, and the obtained compound of formula **(I)** can be purified by chromatography, for example by chromatography on silica gel.

In addition, the compound of formula **(I)** can be recrystallized to further increase the degree of purity.

For example, the compound of formula **(I)** can be recrystallized from toluene.

The inventors of the present invention surprisingly found that the compound of formula **(I)** prepared according to the process of the present invention has a chemical purity, evaluated by HPLC, equal to or greater than 98,0% (Area %), for instance between 98.0% and 100.0% (Area %), equal to 99.0% or between 99,0% and 100,0% (Area %), or equal to 99.5% or between 99,5% and 100,0% (Area %), preferably equal to or greater than 99.0%, more preferably equal to or greater than 99.5%, and where each impurity is typically present in a percentage equal to or less than 0.1%, for example in a percentage between 0.1% and 0.00001% (100 ppb), or in in a percentage between 0.05% and 0.0001% (1 ppm), or in a percentage equal to or less than 0.05%, preferably equal to or less than 0.03%, more preferably equal to or less than 0.01%.

Thus, a compound of formula **(I)**, prepared according to the present invention, is a product, which is particularly suitable as a high quality intermediate for preparing APIs (Active Pharmaceutical Ingredients), which are active particularly as agents for the treatment of diseases associated to the central nervous system (CNS). Examples include, but are not limited to, amitriptyline, nortriptyline or noxiptilina.

The compound of formula **(I)** prepared according to the present invention can be converted into amitriptyline, nortriptyline or noxiptiline respectively, according to known methods.

For example, amitriptyline of formula **(IV)**, or a pharmaceutically acceptable salt thereof, 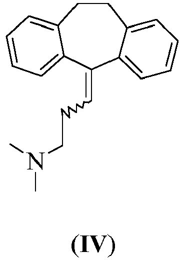 can be obtained by a process comprising reacting the compound of formula **(I)** 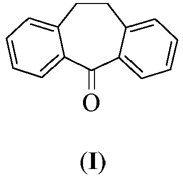 with 3-dimethylaminopropylmagnesium chloride of formula **(V)** 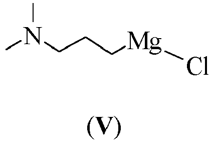 to give the corresponding tertiary alcohol of formula **(VI)** 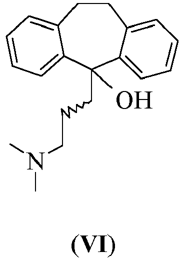 and subsequently treating the compound of formula **(VI)** with an acid, for example as reported by Merck & C. Inc. in BE 584061.

According to the present invention, the term "pharmaceutically acceptable salts" comprise conventional non-toxic salts obtained by salification with inorganic acids (e.g. hydrochloric, hydrobromide, sulphuric or phosphoric acids), or with organic acids (e.g. acetic, propionic, succinic, benzoic, cinnamic, mandelic, salicylic, glycolic, lactic, oxalic, malic, maleic, malonic, fumaric, pamoic, tartaric, citric, p-toluenesulfonic or methanesulfonic acids).

In a preferred aspect of the invention, the pharmaceutically acceptable salt is the hydrochloride salt.

3-Dimethylaminopropylmagnesium chloride of formula **(V)** is a known compound or can be prepared according to methods well known to the skilled person in the art, for instance by treating 3-dimethylaminopropylchloride with magnesium. The compound of formula **(V)** is also sold by ABCR (catalogue number: AB491244).

The so obtained amitriptyline of formula **(IV)**, or a pharmaceutically acceptable salt thereof, 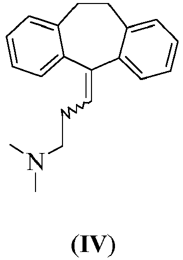 can be further converted into nortriptyline of formula **(VII)** or a pharmaceutically acceptable salt thereof, 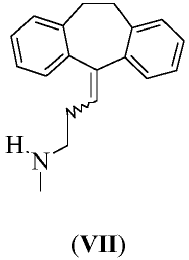 wherein the pharmaceutically acceptable salt is defined as above.

For example, nortriptyline of formula **(VII)** or a pharmaceutically acceptable salt thereof, 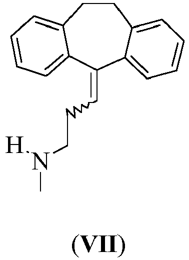 can be obtained by a process comprising
treating amitriptyline of formula **(IV)**, or a pharmaceutically acceptable salt thereof, 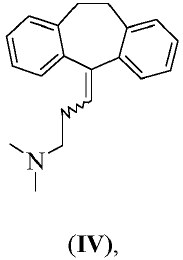 with methyl iodide and then methylamine; or
demethylating amitriptyline of formula **(IV)**, or a pharmaceutically acceptable salt thereof, 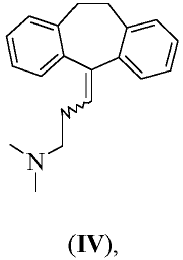
for instance with ethyl chloroformate and a base; or
by treating the compound of formula **(I)** 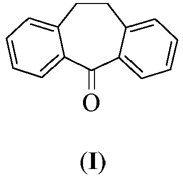
with 3-methylaminopropylmagnesium chloride of formula **(VIII)** 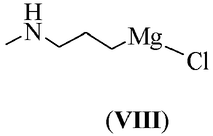
to give the corresponding tertiary alcohol of formula **(IX)** 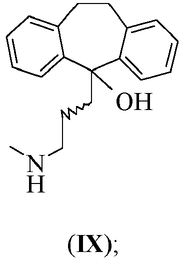
and subsequently treating the compound of formula **(IX)** with an acid.

For example, noxiptiline of formula **(X)**, or a pharmaceutically acceptable salt thereof, 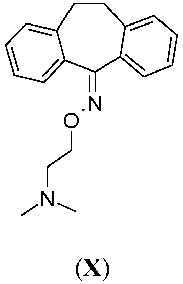 can be prepared by reacting the compound of formula **(I)** first with hydroxylamine, or a salt thereof, and then with 3-chloro-N,N-dimethyl-propan-1-amine.

The pharmaceutically acceptable salt is as defined as above.

A further object of the present invention is directed to a process for preparing amitriptyline, nortriptyline or noxiptiline, comprising the use of a compound of formula **(I)** as a starting product obtained according to the process of the present invention.

In a preferred aspect of the invention, amitriptyline, nortriptyline or noxiptiline have a purity of more than 99.5%, more preferably more than 99.8%.

The following examples further illustrate, but do not limit, the invention.

### Example 1 - Synthesis of Dibenzosuberone of Formula (I)

60 µL of thionyl chloride (97.3 mg, 0.82 mmol) are added to a solution of 56 mg of 2-(phenethyl)benzoic acid of formula **(II)** (0.25 mmol) in 1 mL of para-xylene at room temperature. After 30 minutes, 20 mg of Amberlyst 15^{®} resin (0.09 mmol) are added and the mixture is heated to a temperature of 120°C. After about 24 hours, the mixture is cooled down to room temperature and the resin is filtered off. The organic phase is washed with 1 mL of a 2 M aqueous solution of potassium carbonate and concentrated to residue providing 40 mg of dibenzosuberone of formula **(I)** (yield of 77%).

¹H-NMR (400 MHz, CDCl₃) δ 8.04 (dd, *J* = 7.8, 1.4 Hz, 2H), 7.46 (td, *J* = 7.4, 1.5 Hz, 2H), 7.35 (td, *J* = 7.7, 1.3 Hz, 2H), 7.31 - 7.19 (m, 2H), 3.23 (s, 4H).

### Example 2 - Synthesis of Dibenzosuberone of Formula (I)

20 mg of Amberlyst 15^{®} resin (0,09 mmol) are added to a solution of 56 mg of 2-(phenethyl)benzoic acid of formula **(II)** (0,25 mmol) in 1 mL para-xylene at room temperature, and the mixture is heated to a temperature of 120°C. After about 24 hours, an NMR check of the reaction mixture is carried out, observing a conversion of the 8% dibenzosuberone of formula **(I)**. It is noted that the desired product is formed, but more slowly than in the presence of an activating agent as defined above.

¹H-NMR (400 MHz, CDCl₃) δ 8.04 (dd, *J* = 7.8, 1.4 Hz, 2H), 7.46 (td, *J* = 7.4, 1.5 Hz, 2H), 7.35 (td, *J* = 7.7, 1.3 Hz, 2H), 7.31 - 7.19 (m, 2H), 3.23 (s, 4H). Comparative Examples

### 3 and 4 - Synthesis of Dibenzosuberone of Formula (I) with Nafion-H^{®} Resin

The procedures of examples 1 and 2 are repeated using a Nafion-H^{®} resin instead of the Amberlyst 15^{®} resin. The Nafion-H^{®} resin was purchased from Merck (catalogue code: 676470, batch number: MKCH2387). Since Olah *et al.* made use of resins produced in house, no direct comparison with their Nafion-H^{®} resin and the experiments performed by Olah *et al.* in *Synlett* and *J*. *Org. Chem.* could be made.

After 24 hours at 120°C in para-xylene the desired product is isolated with the following yields:

**Table 1**

| **Experiment** | **Activating agent** | **Yield [%]** |
|---|---|---|
| **3** | - | 3 |
| **4** | SOCl₂ | 2 |

It becomes immediately evident that the yields of the desired product obtained by the use of the commercial perfluorinated polymer resin are significantly lower than the yields obtained in those experiments, wherein the process is catalyzed with the copolymer of divinylbenzene-styrenesulfonic acid. Furthermore, more unwanted impurities are formed in the proceedings with the perfluorinated polymer resins.

In addition, the addition of SOCl₂ as an activating agent to the reaction mixture with the Nafion-H^{®} resins decreases the yield of dibenzosuberone of formula (**I)** leading to the formation of several unwanted products, as already disclosed by Olah et al. in Synlett 1999, 7, 1067-1068.

### Example 5 - Synthesis of Dibenzosuberone of Formula (I)

The procedure of Example 1 with the Amberlyst 15^{®} resin was repeated by varying the equivalents SOCl₂, solvents, reaction temperatures, and reaction times:

**Table 2**

| **Experiment** | **SOCl₂ equivalents** | **solvent** | **T [°C]** | **t [h]** | **Yield [%]** |
|---|---|---|---|---|---|
| a | 3.3 | heptane | 100 | 24 | 100 |
| b | 3.3 | toluene | 100 | 24 | 100 |
| c | 3.3 | toluene | 80 | 24 | 100 |
| d | 2 | toluene | 100 | 8 | 63 |
| e | 2 | toluene | 100 | 24 | 79 |

As can be seen from Table 2, the compound of formula **(II)** converts into dibenzosuberone of formula **(I)** in very high yields (in some cases reaching 100%) in a wide range of different solvents, reaction temperatures and with various equivalents SOCl₂.

## Claims

1. A process for preparing a compound of formula **(I)** 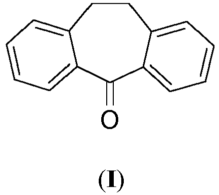 comprising cyclizing the compound of formula **(II)** 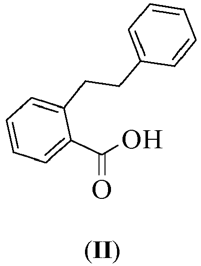 in presence of a sulfonated styrene-divinylbenzene copolymer ion exchange resin.

2. The process according to claim 1, wherein the process is carried out in a solvent.

3. The process according to claim 2, wherein the solvent is an aprotic organic solvent having a boiling temperature between 0°C and 160°C.

4. The process according to claim 3, wherein the solvent is chosen from a linear, branched or cyclic C₄-C₁₂ alkane, an aromatic hydrocarbon, a chlorinated solvent, an ether, a dipolar aprotic solvent, or a mixture of two or more of them.

5. The process according to claim 4, wherein the solvent is chosen from hexane, heptane, cyclohexane, toluene, ortho-xylene, meta-xylene, para-xylene or a mixture of two or more of them.

6. The process according to claims 1 to 5, wherein 100.0 to about 0.001 moles of sulphonic acid groups of the sulfonated styrene-divinylbenzene copolymer are used per mole of compound of formula **(II)**.

7. The process according to claims 1 to 6, wherein the temperature is between 0°C and 160°C.

8. The process according to claims 1 to 7, wherein the process is carried out by a continuous flow process, for instance in a packed bed reactor, or by a batch process.

9. The process according to claims 1 to 8, comprising
activating the compound of formula **(II)** to form a compound of formula **(III)** 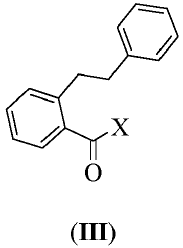
wherein **X** is a halogen or a O-(C=O)-**R** group,
and wherein **R** is chosen from C₁-C₆ alkyl, C₃-C₈ cycloalkyl, C₁-C₆ alkoxy or -O-(C=O)-imidazolyl,
and then treating the compound of formula **(III)** with the sulfonated styrene-divinylbenzene copolymer ion exchange resin.

10. The process according to claim 9, wherein **X** is chlorine.

11. The process according to claims 9 or 10, wherein the process is carried out using from 1.1 to 5.0 moles, for example 2.0 moles, 2.5 moles, 2.7 moles, 3.0 moles, 3.3 moles, 3.5 moles, 3.7 moles or 4.0 moles of the activating agent per mole of the compound of formula **(II)**.

12. The process according to claims 1 to 11, further comprising preparing amitriptyline, nortriptyline or noxiptiline.

13. The process according to claim 12, comprising preparing amitriptyline of formula **(IV)**, or a pharmaceutically acceptable salt thereof, 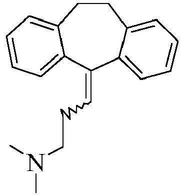 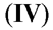 comprising reacting the compound of formula **(I)** 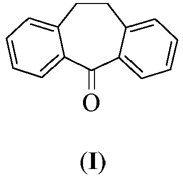 with 3-dimethylaminopropylmagnesium chloride of formula **(V)** 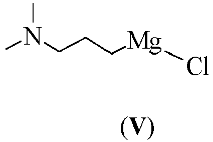 to give the corresponding tertiary alcohol of formula **(VI)** 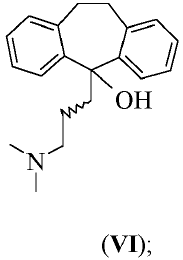 and subsequently treating the compound of formula **(VI)** with an acid.

14. The process according to claims 12 or 13, comprising the preparation of nortriptyline of formula **(VII)** or a pharmaceutically acceptable salt thereof, 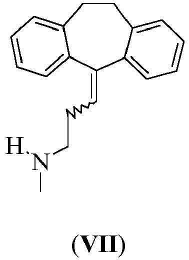
comprising reacting amitriptyline of formula **(IV)**, or a pharmaceutically acceptable salt thereof, 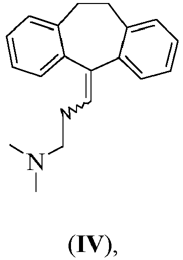
with methyl iodide and then methylamine; or
demethylating amitriptyline of formula **(IV)**, or a pharmaceutically acceptable salt thereof, 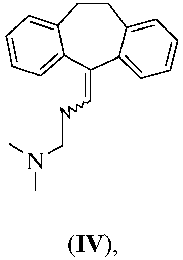
with ethyl chloroformate and a base; or
by treating the compound of formula **(I)** 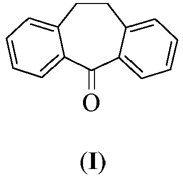
with 3-methylaminopropylmagnesium chloride of formula **(VIII)** 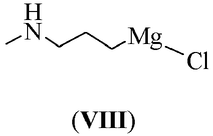
to give the corresponding tertiary alcohol of formula **(IX)** 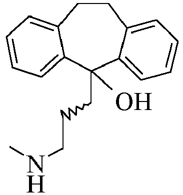 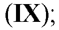 and subsequently treating the compound of formula **(IX)** with an acid.

15. The process according to claim 12, comprising preparing noxiptiline of formula **(X)**, or a pharmaceutically acceptable salt thereof, 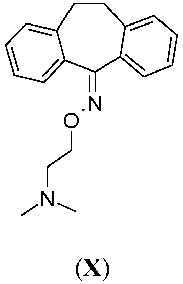 by reacting the compound of formula **(I)** first with hydroxylamine, or a salt thereof, and then with 3-chloro-N,N-dimethyl-propan-1-amine.

## Patentansprüche

1. Verfahren zur Herstellung einer Verbindung der Formel **(I)** 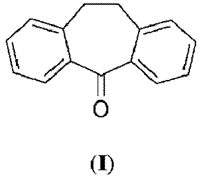 umfassend die Cyclisierung der Verbindung der Formel **(II)** 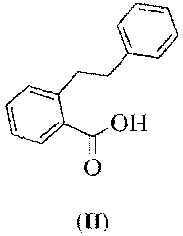 in Gegenwart eines sulfonierten Styrol-Divinylbenzol-Copolymer-Ionenaustauschharzes.

2. Verfahren nach Anspruch 1, wobei das Verfahren in einem Lösungsmittel durchgeführt wird.

3. Verfahren nach Anspruch 2, wobei das Lösungsmittel ein aprotisches organisches Lösungsmittel mit einer Siedetemperatur zwischen 0°C und 160°C ist.

4. Verfahren nach Anspruch 3, wobei das Lösungsmittel ausgewählt ist aus einem linearen, verzweigten oder cyclischem C₄-C₁₂-Alkan, einem aromatischen Kohlenwasserstoff, einem chlorierten Lösungsmittel, einem Ether, einem dipolaren aprotischen Lösungsmittel oder einem Gemisch aus zwei oder mehreren davon.

5. Verfahren nach Anspruch 4, wobei das Lösungsmittel ausgewählt ist aus Hexan, Heptan, Cyclohexan, Toluol, ortho-Xylol, meta-Xylol, para-Xylol oder einem Gemisch aus zwei oder mehreren davon.

6. Verfahren nach einem der Ansprüche 1 bis 5, wobei 100,0 bis etwa 0,001 Mol Sulfonsäuregruppen des sulfonierten Styrol-Divinylbenzol-Copolymers pro Mol der Verbindung der Formel **(II)** verwendet werden.

7. Verfahren nach einem der Ansprüche 1 bis 6, wobei die Temperatur zwischen 0°C und 160°C liegt.

8. Verfahren nach einem der Ansprüche 1 bis 7, wobei das Verfahren in einem kontinuierlichen Durchflussverfahren, beispielsweise in einem Festbettreaktor, oder in einem Chargenverfahren durchgeführt wird.

9. Verfahren nach einem der Ansprüche 1 bis 8, das Folgendes umfasst:
Aktivieren der Verbindung der Formel **(II)** zur Bildung einer
Verbindung der Formel **(III)** 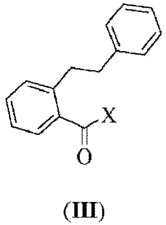
wobei **X** ein Halogen oder eine O-(C=O)-**R**-Gruppe ist,
und wobei **R** ausgewählt ist aus C₁-C₆-Alkyl, C₃-C₈-Cycloalkyl,
C₁-C₆-Alkoxy oder -O-(C=O)-Imidazolyl,
und anschließende Behandlung der Verbindung der Formel **(III)** mit dem sulfonierten Styrol-Divinylbenzol-Copolymer-Ionenaustauschharz.

10. Verfahren nach Anspruch 9, wobei **X** Chlor ist.

11. Verfahren nach Anspruch 9 oder 10, wobei das Verfahren unter Verwendung von 1,1 bis 5,0 Molen, beispielsweise 2,0 Molen, 2,5 Molen, 2,7 Molen, 3,0 Molen, 3,3 Molen, 3,5 Molen, 3,7 Molen oder 4,0 Molen des Aktivierungsmittels pro Mol der Verbindung der Formel **(II)** durchgeführt wird.

12. Verfahren nach einem der Ansprüche 1 bis 11, das ferner die Herstellung von Amitriptylin, Nortriptylin oder Noxiptilin umfasst.

13. Verfahren nach Anspruch 12, umfassend die Herstellung von Amitriptylin der Formel **(IV)** oder eines pharmazeutisch akzeptablen Salzes davon, 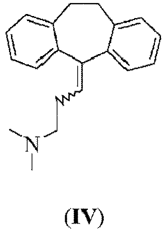 umfassend die Umsetzung der Verbindung der Formel **(I)** 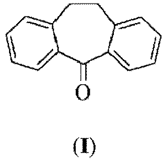 mit 3-Dimethylaminopropylmagnesiumchlorid der Formel **(V)** 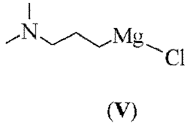 unter Bildung des entsprechenden tertiären Alkohols der Formel **(VI)** 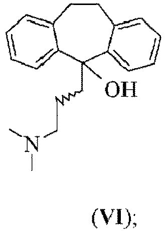 und anschließende Behandlung der Verbindung der Formel **(VI)** mit einer Säure.

14. Verfahren nach Anspruch 12 oder 13, umfassend die Herstellung von Nortriptylin der Formel **(VII)** oder eines pharmazeutisch akzeptablen Salzes davon, 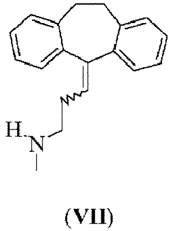 umfassend die Umsetzung von Amitriptylin der Formel **(IV)** oder eines pharmazeutisch akzeptablen Salzes davon, 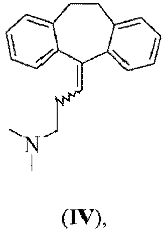 mit Methyliodid und anschließend mit Methylamin; oder Demethylierung von Amitriptylin der Formel **(IV)** oder eines pharmazeutisch akzeptablen Salzes davon, 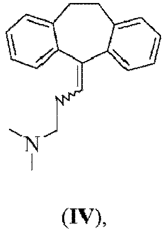 mit Chlorameisensäureethylester und einer Base; oder durch Behandlung der Verbindung der Formel **(I)** 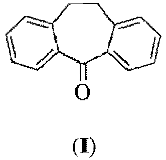 mit 3-Methylaminopropylmagnesiumchlorid der Formel **(VIII)** 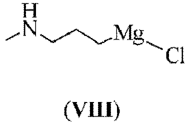 um den entsprechenden tertiären Alkohol der Formel **(IX)** zu erhalten 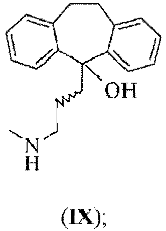 und anschließende Behandlung der Verbindung der Formel **(IX)** mit einer Säure.

15. Verfahren nach Anspruch 12, umfassend die Herstellung von Noxiptilin der Formel **(X)** oder eines pharmazeutisch akzeptablen Salzes davon, 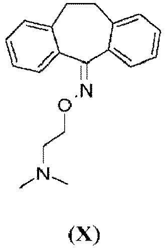 durch Umsetzen der Verbindung der Formel **(I)** zuerst mit Hydroxylamin oder einem Salz davon und dann mit 3-Chlor-N,N-dimethyl-propan-1-amin.

## Revendications

1. Procédé de préparation d'un composé de formule (I) 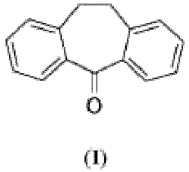 comprenant la cyclisation du composé de formule (II) 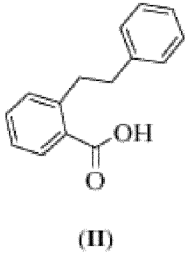 en présence d'une résine échangeuse d'ions à base de copolymère sulfoné de styrène et de divinylbenzène.

2. Procédé selon la revendication 1, dans lequel le procédé est effectué dans un solvant.

3. Procédé selon la revendication 2, dans lequel le solvant est un solvant organique aprotique ayant une température d'ébullition comprise entre 0 °C et 160 °C.

4. Procédé selon la revendication 3, dans lequel le solvant est choisi parmi un alcane en C₄-C₁₂ linéaire, ramifié ou cyclique, un hydrocarbure aromatique, un solvant chloré, un éther, un solvant aprotique dipolaire ou un mélange de deux ou plus d'entre eux.

5. Procédé selon la revendication 4, dans lequel le solvant est choisi parmi l'hexane, l'heptane, le cyclohexane, le toluène, l'orthoxylène, le métaxylène, le paraxylène ou un mélange de deux ou plus d'entre eux.

6. Procédé selon les revendications 1 à 5, dans lequel 100,0 à environ 0,001 moles de groupes acide sulfonique du copolymère sulfoné de styrène et de divinylbenzène sont utilisées par mole de composé de formule (II).

7. Procédé selon les revendications 1 à 6, dans lequel la température est comprise entre 0 °C et 160 °C.

8. Procédé selon les revendications 1 à 7, dans lequel le procédé est effectué par un procédé continu, par exemple un réacteur à lit à garnissage, ou par un procédé discontinu.

9. Procédé selon les revendications 1 à 8, comprenant l'activation du composé de formule (II) pour former un composé de formule (III) 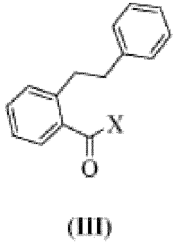
dans lequel X est un halogène ou un groupe O-(C=O)-R,
et dans lequel R est choisi parmi l'alkyle en C₁-C₆, le cycloalkyle en C₃-C₈, l'alcoxy en C₁-C₆ ou le -O-(C=O)-imidazolyle,
et puis le traitement du composé de formule (III) avec la résine échangeuse d'ions à base de copolymère sulfoné de styrène et de divinylbenzène.

10. Procédé selon la revendication 9, dans lequel X est du chlore.

11. Procédé selon les revendications 9 ou 10, dans lequel le procédé est effectué en utilisant de 1,1 à 5,0 moles, par exemple 2,0 moles, 2,5 moles, 2,7 moles, 3,0 moles, 3,3 moles, 3,5 moles, 3,7 moles ou 4,0 moles de l'agent activant par mole du composé de formule (II).

12. Procédé selon les revendications 1 à 11, comprenant en outre la préparation d'amitriptyline, de nortriptyline ou de noxiptiline.

13. Procédé selon la revendication 12, comprenant la préparation d'amitriptyline de formule (IV) ou d'un sel pharmaceutiquement acceptable de celle-ci, 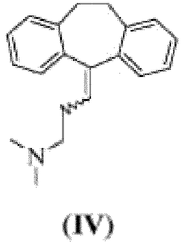
comprenant la réaction du composé de formule (I) 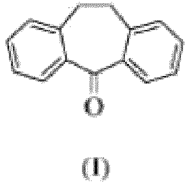
avec du chlorure de 3-diméthylaminopropylmagnésium de formule (V) 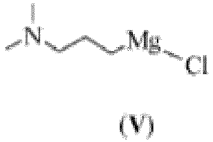
pour donner l'alcool tertiaire correspondant de formule (VI) 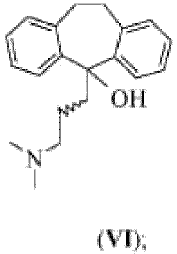 et puis le traitement du composé de formule (VI) avec un acide.

14. Procédé selon les revendications 12 ou 13, comprenant la préparation de nortriptyline de formule (VII) ou d'un sel pharmaceutiquement acceptable de celle-ci, 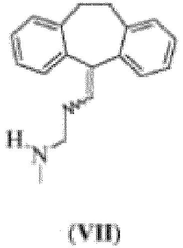
comprenant la réaction de l'amitriptyline de formule (IV) ou d'un sel pharmaceutiquement acceptable de celle-ci, 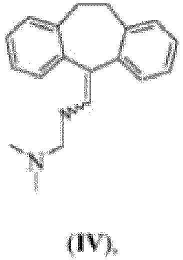
avec de l'iodure de méthyle et puis de la méthylamine ; ou
la déméthylation de l'amitriptyline de formule (IV) ou d'un sel pharmaceutiquement acceptable de celle-ci, 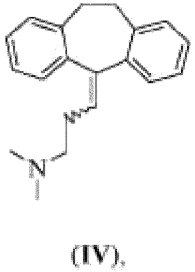
avec du chloroformiate d'éthyle et une base ; ou en traitant le composé de formule (I) 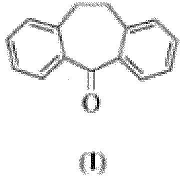
avec du chlorure de 3-méthylaminopropylmagnésium de formule (VIII) 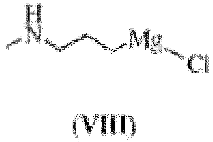
pour donner l'alcool tertiaire correspondant de formule (IX) 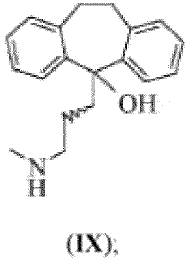
et puis le traitement du composé de formule (IX) avec un acide.

15. Procédé selon la revendication 12, comprenant la préparation de noxiptiline de formule (X) ou d'un sel pharmaceutiquement acceptable de celle-ci, 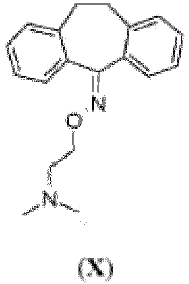 en faisant réagir le composé de formule (1) d'abord avec de l'hydroxylamine, ou un sel de celle-ci, et puis avec de la 3-chloro-N,N-diméthylpropan-1-amine.
